# EUROPEAN PATENT APPLICATION

(11) **EP 1 181 927 A1**
(43) Date of publication of application: **27.02.2002**
(21) Application number: 01912445.2
(22) Date of filing: 16.03.2001
(51) Int. Cl.: A61K 7/48, A61K 7/00, A61P 17/16

(54) **AGENTS IMPROVING SKIN BARRIER FUNCTION**

(30) Priority: 31.03.2000 JP 2000098039
(71) Applicant: SHISEIDO COMPANY LIMITED, Chuo-ku, Tokyo 104-8010 (JP)
(72) Inventor: HIRAO, Tetsuji Shiseido Res. Ctr.(Kanazawa-hakkei), Yokohama-shi, Kanagawa 236-8643 (JP)
(74) Representative: Albrecht, Thomas, Dr.
(86) International application number: JP0102116
(87) International publication number: WO0174326

(57) **Abstract**

Here is disclosed a cosmetic or dermatological preparation containing as an active ingredient a substance which is sugar oil or extract originating in a plant including various crude drugs and shows an action to accelerate formation or maturation of cornified envelope. Such preparation is effective for improving skin barrier functions.

## Description

### Technical Field

This invention relates to the technical fields of cosmetics or dermatological preparations. More detailedly, the invention relates to an agent for accelerating formation or maturation of cornified envelope (hereinafter, abbreviated as CE) and its use as an agent for improving skin barrier functions.

### Background Art

Horny layer is composed of corneocytes formed by terminal differentiation of epidermal keratinocytes and intercellular lipid surrounding them. It has come to be clear that intercellular lipid forms lamella structure using ceramide, cholesterol, fatty acids, etc. as components, and plays an important role in barrier functions of horny layer. This is supported by the fact that intercellular lipid is disordered in its form and composition in various skin diseases wherein barrier functions of horny layer lower and skin troubles such as rough skin. On the other hand, corneocytes are composed of keratin fibers as a main component and CE (cornified envelope) wrapping them. CE is formed by that plural CE precursor proteins produced in epidermal keratinocytes as the cells differentiate are crosslinked with an enzyme transglutaminase and insolubilized. Further, it is suggested that ceramide, etc. are covalently bonded to part of CE to give hydrophobic structure, and thereby the basis of the lamella structure of intercellular lipid is supplied and the basis of horny layer barrier functions is formed.

CE can be prepared by boiling epidermal tissue, cultured skin cells or the like in a solution containing a surfactant such as sodium dodecyl sulfate and a reducing agent such as mercaptoethanol and obtaining the insoluble fraction separated from the soluble components by a means such as centrifugation. The properties and state of CE can be evaluated by morphologically observing it by a microscope. Michel reports that more CE of fragile structure is observed at deeper part of horny layer than at the outmost layer of horny layer (J. Invest. Dermatol. 91: 11-15, 1988). Further, it is reported that fragile CE is observed even at the outmost layer in the case of psoriasis, lamellar ichthyosis, etc. (Br. J. Dermatol. 122: 15-21, 1990).

The present inventors have made sequential researches for elucidating the state of skin, particularly horny layer in view of the properties and state of CE. As a result, they found that it is possible to grasp abnormal change of the barrier functions of horny layer accurately by examining, in addition to morphological observation of CE, for example, the hydrophobicity of CE by Nile Red staining or the antigenicity of involucrin as a constituent by immunofluorescent stain.

They further found that formation or maturation degree of CE in cultured keratinocytes can be evaluated by such stainability. Based on these findings, the present inventors proposed a skin quality-evaluating method (Japanese Patent Application No. 207890/ 1999).

On the other hand, in treatment of dermatological diseases such as psoriasis wherein it is recognized that fragile CE is observed even at the outmost layer and these diseases are accompanied by parakeratosis (or lowering of horny layer barrier functions) in pathological tissue findings, external preparations such as chiefly corticosteroids have hitherto been used, and various measures have also been made against skin troubles such as rough skin caused by lowering of horny layer barrier functions.

Although, as stated above, various measures have been made against parakeratosis (or immatureness of keratinocytes) or lowering of horny layer barrier functions of skin, necessity of providing further effective means still exists. Particularly, provision of a means to accelerate quantitatively and qualitatively formation or maturation of CE which are suggested to form the basis of horny layer barrier functions, will be desired.

### Disclosure of Invention

It has become clear by using a method as mentioned above capable of accurately evaluating the maturation degree of CE that a lot of immature CE exists even in horny layer of the face, etc. wherein a certain skin barrier function lowered, although the horny layer has not come to dermatological disease. The present inventors assumed that if the immature CE can be leaded to sufficient maturation degree, skin barrier functions can surely be improved in proportion thereto.

Under the assumption, they made researches into the ability of various components to accelerate formation or maturation degree of CE and, as a result, confirmed that if sufficient maturation degree of CE is brought about, skin barrier functions can surely be improved. This invention is based on these findings. In addition, various biological products have been found as ones having an action to accelerate formation or maturation of CE for bringing about sufficient maturation degree of CE as mentioned above. The invention is based on these findings.

Therefore, the invention of an embodiment relates to an agent for improving skin barrier functions which comprises a substance having an action to accelerate formation or maturation of cornified envelope (CE) in an amount enough to attain sufficient maturation degree of CE in epidermis, and diluents or carriers.

The invention of another embodiment relates to a method for improving skin barrier functions which comprises topically administering a substance having an action to accelerate formation or maturation of CE onto the skin of a subject, and bringing about sufficient maturation degree of CE in the epidermis of the skin. Further, a method for cosmetic skin care utilizing such improvement is also provided.

The invention of still another embodiment relates to use of a substance having an action to accelerate formation or maturation of CE in an amount enough to attain sufficient maturation degree of CE in epidermis, for preparation of an agent for improving skin barrier functions.

### Best Mode for Carrying out the Invention

Formation or maturation of CE in the invention means that plural CE precursor proteins produced in proportion as epidermal keratinocytes differentiate are crosslinked and insolubilized by action of an enzyme transglutaminase, etc., and further, ceramide, etc. are covalently bonded to part of them to give hydrophobic structure. According to the invention, degree of formation or maturation of CE can be detected and determined by positiveness of staining with a dye capable of selectively staining the hydrophobic region, for example, Nile Red or by lowering or disappearance of antigenicity of proteins constituting CE (specifically, lowering or disappearance of bondability of an antibody against the proteins).

Therefore, "sufficient maturation degree of CE being attained" in the invention means being in such a state that the antigenicity of constitutive proteins of CE, for example involucrin substantially disappeared. "substantially disappeared" means such a state that immature CE is not found in the evaluation of the properties and state of CE described later.

According to the invention, any substances can be used for improvement of skin barrier functions so long as they are substances having an action to accelerate formation or maturation of CE and meeting the objects of the invention. This will be understood from that substances exerting the action can be chosen, for example, from the following various substance groups.

However, there can be mentioned, as such active substances, products originating in plants (herbs, roots, leaves, flowers, fruits, seeds, etc.) usable in the cosmetic field. Plants usable in the cosmetic field can be selected from plants which are used in folk remedies including Japanese and Chinese crude drugs, and plants enumerated in the clause of "Biolobical Products" of "International Cosmetic Dictionary and Handbook", Seventh Edition, 1997, Published by The Cosmetic, Toiletry, and Fragrance Association, Washington, DC. As substances exerting the above-mentioned action, there can be mentioned, although not limited thereto, olive (Olea europaea L.) oil, camellia (Camellia japonica L.) oil, candle nut oil (oil from seed of Aleurites moluccana Wild.), linseed (seed of Linum usitatisimun L.) oil, avocado (Persea americana Mill.) oil, extract of Caryophylli Flos (bud of Syzygium aromaticum Merrill et Perry) or Caryophylli Flos oil, extract of Juniperus communis L., extract of Eucalyptus globulus Labill, extract of Angelica acutiloba Kitagawa, extract of Matricaria chamomilla L., extract of Phellodendri Cortex (bark of Phellodendron amurense Ruprecht), extract of Coicis Semen (substance obtained by removing seedcase, seedcoat, etc. from the fruit of Coix Cacryma-jobi L. var. ma-yuen Stapf), extract of Thea sinensis L. var. assamica Pieqre, extract of Thea sinensis L., extract of Plectranthus japonicas Koidz., extract of Phaseolus radiatus L. var. aurea Prain, extract of Hydrangeae Dulcis Folium (substance obtained by drying in the sun a fermentation product from leaves of Hydrangea macrophylla Seringe var. thunbergii Makino) and extract of Swertiae Herb (whole plant of Swertia japonica Makino). Although, in the above, the suitable active ingredients are defined as to their origin by specific species, specific mutants or the like, ".....oil" or ".....extract" referred to in the invention also includes ingredients originating in all plants closely related to the above specific species or specific mutants so long as they are obtained from the closely related plants and meet the objects of the invention, and also include those being in any purification stages. As such oils or extracts, those available on the market as crude drugs or raw materials for crude drugs can be used as such in most cases, or after simple treatment (e.g., solvent extraction, distillation, steam distillation, etc.). It is known that various compounds are contained in the above oils or extracts, and they do not always have mutually common chemical structures (see, for example, Hirokawa Yakuyo Shokubutsu Daijiten (Hirokawa Encyclopedia of Medicinal Plants), published by Hirokawa Shoten, February 25, 1980). Nevertheless, substances showing an action to accelerate formation or maturation of CE in the evaluation system established by the present inventors have an effect to improve skin barrier functions in common.

The above substances themselves can be active ingredients for accelerating in vitro or in vivo formation or maturation of CE in keratinocytes or epidermis, and attaining sufficient maturation degree. Therefore, the invention not only ultimately leads to improvement of skin barrier functions, but can contribute to prophylaxis or treatment of dermatological diseases and cosmetic skin care, and, on the other hand, in vitro use can contribute, for example, to provision of epidermic cell preparations.

The above extract or oil is used, as an active ingredient of an improving agent or an accelerating agent according to the invention, generally, as dry weight, in an amount of 0.00001 to 10 % by weight, preferably 0.0001 to 5 % by weight per weight of the total composition. At lower than 0.00001 % by weight, the effects of the invention are hard to exert sufficiently, and even if it is compounded in an amount more than 10 % by weight, so much enhancement of the effects is not attained and formulation becomes undesirably harder.

A composition to be thus prescribed can be prepared by mixing or homogenizing the above extract or oil into pure water, deionized water or buffered water, a lower alkanol such as methanol, ethanol or isopropyl alcohol or an aqueous solution thereof, glycerol or an aqueous solution thereof, a glycol such as propylene glycol or 1,3-butylene glycol or an aqueous solution thereof, or an oil such as hardened castor oil, vaseline or squalane, if necessary with use of a surfactant or the like. Into the composition of the invention can further appropriately be compounded, in such a range that the effects of the invention are not spoiled, other components usually used for external preparations such as cosmetics or pharmaceuticals, for example whitening agents, humectants, antioxidants, oily substances, ultraviolet absorbers, surfactants, thickeners, higher alcohols, powdery substances, colorants, aqueous substances, water, various skin nutrients, etc., according to necessity. Further, into the composition of the invention can appropriately be compounded sequestering agents such as disodium edetate, trisodium edetate, sodium citrate, sodium polyphosphate, sodium metaphosphate and gluconic acid, drugs such as caffeine, tannin, verapamil, tranexamic acid and its derivatives, grabridin, extract of fruit of Chinese quince with hot water, various crude drugs other than the above-mentioned, tocopherol acetate, and glycylrrhetinic acid and its derivatives or salts, whiteners such as vitamin C, magnesium ascorbate phosphate, ascorbic acid glucoside, arbutin and kojic acid, saccharides such as glucose, fructose, mannose, sucrose and trehalose, vitamin A derivatives such as retinoic acid , retinol, retinol acetate and retinol palmitate, etc.

As to the agent or composition according to the invention, its dosage form is not particularly limited, and can be any dosage forms such as solutions, solubilizing forms, emulsified forms, dispersed powders, water-oil two layer forms, water-oil-powder three layer forms, ointments, gels or aerosols. Its use form can also be optional, and can, for example be facial cosmetics such as skin lotion, liquid cream, cream and pack, foundation, and further makeup cosmetics, cosmetics for hair, aromatic cosmetics, bathing agents, etc., but is not limited thereto.

When an agent according to the invention is used on a living body, a composition as mentioned above is endermically administered to local skin or the whole body skin of a subject. Its dose cannot be limited because the optimal amount varies depending on the age, sex and skin state of subjects, but, usually, it is sufficient that a composition prepared as mentioned above is administered onto the skin once or several times a day. If necessary, the dose or administration frequency can be determined referring to results obtained by evaluating a suitable specimen according to the evaluation method described later.

The invention is further detailedly described below referring to specific examples, but these examples are provided for the purpose of facilitating the understanding of the invention. In the following description, percentages (%) are based on weight/weight, unless otherwise defined.

### Example 1: Tests of formation of CE and evaluation of properties and state of CE

### Formation of CE

Human epidermal keratinocytes were cultured according to the method of Rheinwald & Green (Cell 6: 331-344, 1975). The cells were cultured in a growth medium (DMEM-Ham's F12 (3 : 1) containing 10 % fetal bovine serum, 0.5 µg/ml hydrocortisone, 5 µg/ml insulin, 10⁻¹⁰ M cholera toxin, 10 ng/ml epidermal growth factor and 1.8 × 10⁻⁴ M adenine) up to confluent, the medium was replaced with a medium (MEM containing 0.1 % bovine serum albumin) containing a test substance, and culturing was continued for further 1 week. After completion of the culturing, Tris-hydrochloric acid buffer containing dithiothreitol and sodium dodecyl sulfate was added, and the mixture was heated at 100°C for 10 minutes. The insoluble substance was collected by centrifugation at 4,000 g for 10 minutes. The eluent addition and heating were repeated and thereby soluble components were thoroughly removed. The thus obtained insoluble substance was referred to as CE. The amount of the obtained CE was evaluated by visual observation. Otherwise, the number was counted under a microscope using a cytometer. The results are shown in Table 1.

### Evaluation of the properties and state of CE

CE prepared by the above method was added dropwise onto slide glass, air-dried, and fixed in cold acetone. The fixed substance was hydrated with Dulbecco's phosphate-buffered physiological saline solution, and murine anti-human involucrin antibody (NOVOCASTRA Co.) was reacted as a primary antibody. The excess antibody was removed by washing, and FITC-labeled rabbit anti-murine immunoglobulin antibody was reacted as a second antibody. The excess antibody was removed by washing, Nile Red staining solution was reacted, and the reaction mixture was sealed and observed by a fluorescence microscope. The observed image was taken into a computer through a CCD camera, the maturation degree of CE was evaluated using an image-analyzing software (Mac Scope) wherein involucrin positiveness shows the CE being immature and Nile Red positiveness shows the CE being mature. The results are shown in Table 1.

The evaluation shown in Table 1 was made based on the following criteria.
CE formation-accelerating effect:
- - :: No CE formation
- + :: CE formation is observed, but a little less than in positive control (growth medium)
- ++ :: CE formation in an amount equal to that in positive control (growth medium) is observed
- +++:: Clearly more CE formation than in positive control (growth medium) is observed
Evaluation of CE maturation degree:
- - :: involucrin positive immature CE is observed
- + :: involucrin positive immature CE is not observed

**Table 1**

| CE formation-accelerating effect | | | | |
|---|---|---|---|---|
| Test substance | Concentration | CE formation-accelerating effect | CE maturation degree | Note |
| Growth medium (a) | | ++ | - | |
| No addition (b) | | - | | |
| Olive oil | 3% | + | + | (c) |
| Extra virgin olive oil | 1% | + + | + | (c) |
| | 3% | +++ | + | |
| Linseed oil | 3% | ++ | + | (d) |
| Camellia oil | 3% | +++ | + | (c) |
| Candle nut oil | 3% | +++ | + | (c) |
| Avocado oil | 3% | + | + | (c) |
| | | | | |
| Ethanol (solvent control) | 0.3% | - | | |
| Extract of Caryophylli Flos | 15 µg/ml | ++ | + | (e) |
| Extract of Juniperus communis L. | 15 µg/ml | ++ | + | (f) |
| Extract of Eucalyptus globulus Labill | 15 µg/ml | ++ | + | (e) |
| Extract of Phellodendri Cortex | 15 µg/ml | ++ | + | (e) |
| Extract of Scutellariae Radix | 15 µg/ml | ++ | + | (e) |
| Extract of Coicis Semen | 15 µg/ml | ++ | + | (e) |
| Extract of Thea sinensis L. var. assamica Pieqre | 15 µg/ml | ++ | + | (e) |
| Extract of Thea sinensis L. | 15 µg/ml | + + | + | (e) |
| Extract of Plectranthus japonicas Koidz | 15 µg/ml | ++ | + | (e) |
| Extract of Phaseolus radiatus L. var. aurea Prain | 15 µg/ml | + + | + | (e) |
| Extract of Hydrangeae Dulcis Folium | 15 µg/ml | ++ | + | (e) |
| | | | | |
| Squalane | 3% | - | | |
| Extract of Angelica acutiloba Kitagawa with squalane | 3% | ++ | + | (e) |
| Extract of Matricaria chamomilla L. with squalane | 3% | ++ | + | (e) |
| (a) The same as the above-mentioned growth medium | | | | |
| (b) MEM containing 0.1 % bovine serum albumin | | | | |
| (c) Product meeting the Cosmetic Compounding Standard | | | | |
| (d) Obtained from Wako Pure Chemical Industries, Ltd. | | | | |
| (e) Dry residue obtained by distilling off the solvent from a product meeting the Cosmetic Compounding Standard was used in the test. | | | | |
| (f) Obtained from Maruzen Pharmaceuticals Co., Ltd. | | | | |

### Formulation Example 1: Liquid cream

### (Prescription)

| | % by weight |
|---|---|
| Stearic acid | 2.5 |
| Cetyl alcohol | 1.5 |
| Vaseline | 5.0 |
| Liquid paraffin | 5.0 |
| Olive oil (extra virgin) | 5.0 |
| Polyoxyethylene (10 moles) monooleate ester | 2.0 |
| Polyethylene glycol 1500 | 3.0 |
| Triethanolamine | 1.0 |
| Carboxyvinyl polymer | 0.05 |
| Sodium hydrogensulfite | 0.01 |
| Ethylparaben | 0.2 |
| Perfume | suitable amount |
| Deionized water | balance |

### (Preparation process)

Carboxyvinyl polymer was dissolved in a small amount of deionized water (phase A). Polyethylene glycol 1500 and triethanolamine were added to the residual deionized water, and the mixture was heated to make a solution and held at 70°C (aqueous phase). The other components were mixed and melt with heating, and held at 70°C (oil phase). The oil phase was added to the aqueous phase and preliminary emulsification were made, and the phase A was added. The mixture was uniformly emulsified by a homomixer, and after the emulsification, cooled to 30°C under sufficient stirring to obtain a liquid cream.

### Formulation Example 2: Skin lotion

### (Prescription)

| | % by weight |
|---|---|
| Ethanol | 5.0 |
| Carboxyvinyl polymer | 0.3 |
| Polyoxyethylene (15 moles) oleyl ether | 0.8 |
| Glycerol | 5.0 |
| 1,3-Butylene glycol | 5.0 |
| Extract of Caryophylli Flos | 1.0 |
| Citric acid | 0.03 |
| Sodium citrate | 0.07 |
| Methylparaben | 0.1 |
| Deionized water | balance |

### (Preparation process)

Citric acid and sodium citrate were dissolved in deionized water (aqueous phase). The other components were mixed and stirred to give a solution. The solution was added to the aqueous phase, and the mixture was homogenized to obtain a skin lotion.

### Formulation Example 2: Cream

### (Prescription)

| | % by weight |
|---|---|
| Stearic acid | 2.0 |
| Stearyl alcohol | 7.0 |
| Hydrogenated lanolin | 2.0 |
| Extract of Angelica acutiloba Kitagawa with squalane | 5.0 |
| 2-Octyldodecyl alcohol | 6.0 |
| Polyoxyethylene (25 moles) cetyl alcohol ether | 3.0 |
| Glycerol monostearate ester | 2.0 |
| 1,3-Butylene glycol | 5.0 |
| Trisodium edetate | 0.1 |
| Sodium glycyrrhetinate | 0.1 |
| Vitamin E acetate | 0.3 |
| Ethylparaben | 0.3 |
| Deionized water | balance |

### (Preparation process)

1,3-Butylene glycol, trisodium edetate and sodium glycyrrhetinate were added to deionized water, and the mixture was held at 70°C (aqueous phase). On the other hand, the other components were mixed and melt with heating, and held at 70°C (oil phase). The oil phase was added to the aqueous phase, and the mixture was preliminarily emulsified, uniformly emulsified by a homomixer, and cooled to 30°C to obtain a cream.

### Industrial Applicability

As stated above, according to the invention, a means capable of attaining sufficient maturation degree of CE in epidermis can be provided. Therefore, the invention can be utilized in the cosmetic field or cosmetic industry.

## Claims

1. An agent for improving skin barrier functions which comprises a substance having an action to accelerate formation or maturation of cornified envelope (CE) in an amount enough to attain sufficient maturation degree of CE in epidermis, and diluents or carriers.

2. The agent according to claim 1 wherein the substance having an action to accelerate formation or maturation of CE is a product originating in a plant usable in the cosmetic field.

3. The agent according to claim 1 wherein the substance having an action to accelerate formation or maturation of CE is one substance or a mixture of two or more selected from the group consisting of olive oil, camellia oil, candle nut oil, linseed oil, avocado oil, extract of Caryophylli Flos, extract of Juniperus communis L., extract of Eucalyptus globulus Labill, extract of Angelica acutiloba Kitagawa, extract of Matricaria chamomilla L., extract of Phellodendri Cortex, extract of Scutellariae Radix, extract of Coix Cacryma-jobi L. var. ma-yuen Stapf (Coicis Semen), extract of Thea sinensis L. var. assamica Pieqre, extract of Thea sinensis L., extract of Plectranthus japonicas Koidz., extract of Phaseolus radiatus L. var. aurea Prain, extract of Hydrangeae Dulcis Folium and extract of Swertia japonica Makino.

4. An agent for accelerating in vivo or in vitro formation or maturation of cornified envelope which comprises one substance or a mixture of two or more selected from the group consisting of olive oil, camellia oil, candle nut oil, linseed oil, avocado oil, extract of Caryophylli Flos, extract of Juniperus communis L., extract of Eucalyptus globulus Labill, extract of Angelica acutiloba Kitagawa, extract of Matricaria chamomilla L., extract of Phellodendri Cortex, extract of Scutellariae Radix, extract of Coix Cacryma-jobi L. var. ma-yuen Stapf (Coicis Semen), extract of Thea sinensis L. var. assamica Pieqre, extract of Thea sinensis L., extract of Plectranthus japonicas Koidz., extract of Phaseolus radiatus L. var. aurea Prain, extract of Hydrangeae Dulcis Folium and extract of Swertia japonica Makino, and diluents or carriers.

5. A method for improving skin barrier functions which comprises preparing a substance having an action to accelerate formation or maturation of CE, topically administering the substance onto the skin of a subject who is fit to be treated with the substance, and bringing about sufficient maturation degree of CE in the epidermis of the skin.

6. The method according to claim 5 wherein the substance having an action to accelerate formation or maturation of CE is a product originating in a plant usable in the cosmetic field.

7. The method according to claim 5 wherein the substance having an action to accelerate formation or maturation of CE is one substance or a mixture of two or more selected from the group consisting of olive oil, camellia oil, candle nut oil, linseed oil, avocado oil, extract of Caryophylli Flos, extract of Juniperus communis L., extract of Eucalyptus globulus Labill, extract of Angelica acutiloba Kitagawa, extract of Matricaria chamomilla L., extract of Phellodendri Cortex, extract of Scutellariae Radix, extract of Coix Cacryma-jobi L. var. ma-yuen Stapf (Coicis Semen), extract of Thea sinensis L. var. assamica Pieqre, extract of Thea sinensis L., extract of Plectranthus japonicas Koidz., extract of Phaseolus radiatus L. var. aurea Prain, extract of Hydrangeae Dulcis Folium and extract of Swertia japonica Makino.

8. A method for cosmetic skin care which comprises preparing a substance having an action to accelerate formation or maturation of CE, topically administering the substance onto the skin of a subject who is fit to be treated with the substance, and bringing about sufficient maturation degree of CE in the epidermis of the skin and thereby improving skin barrier functions.

9. The method according to claim 8 wherein the substance having an action to accelerate formation or maturation of CE is one substance or a mixture of two or more selected from the group consisting of olive oil, camellia oil, candle nut oil, linseed oil, avocado oil, extract of Caryophylli Flos, extract of Juniperus communis L., extract of Eucalyptus globulus Labill, extract of Angelica acutiloba Kitagawa, extract of Matricaria chamomilla L., extract of Phellodendri Cortex, extract of Scutellariae Radix, extract of Coix Cacryma-jobi L. var. ma-yuen Stapf (Coicis Semen), extract of Thea sinensis L. var. assamica Pieqre, extract of Thea sinensis L., extract of Plectranthus japonicas Koidz., extract of Phaseolus radiatus L. var. aurea Prain, extract of Hydrangeae Dulcis Folium and extract of Swertia japonica Makino.

10. Use of a substance having an action to accelerate formation or maturation of CE in an amount enough to attain sufficient maturation degree of CE in epidermis, for preparation of an agent for improving skin barrier functions.
